# EUROPEAN PATENT APPLICATION

(11) **EP 3 744 858 A1**
(43) Date of publication of application: **02.12.2020**
(21) Application number: 18882701.8
(22) Date of filing: 05.09.2018
(51) Int. Cl.: C12Q 1/6886, G01N 33/68, G01N 33/72, G01N 33/574, C12N 15/113

(54) **COMPOSITION FOR DETECTING ESOPHAGEAL CANCER AND USE THEREOF**

(30) Priority: 01.12.2017 CN 201711248825; 28.08.2018 CN 201810989986
(71) Applicant: Biochain (Beijing) Science & Technology, Inc., Beijing 100176 (CN)
(72) Inventor: MA, Jun, Beijing 100176 (CN); HAN, Xiaoliang, Beijing 100176 (CN); WANG, James Jianming, Beijing 100176 (CN); FAN, Daiming, Beijing 100176 (CN); NIE, Yongzhan, Beijing 100176 (CN); HU, Sijuan, Beijing 100176 (CN)
(74) Representative: Lavoix
(86) International application number: PCT/CN2018/104076
(87) International publication number: WO 2019/105090

(57) **Abstract**

Provided are a composition for detecting esophageal cancer and use thereof, the composition comprising a nucleic acid for detecting the methylation status of a target gene, wherein the target gene is one or both of MT1A gene and EPO gene. Also provided is a composition for detecting esophageal cancer, the composition comprising a nucleic acid for detecting the methylation status of a target gene, and an antibody for detecting the concentration of a target protein; wherein the target gene is one or both of MT1A gene and EPO gene, and the target protein is SNCG. Also provided are a test kit comprising said composition, and the use of the composition in the preparation of a test kit for in-vitro detecting esophageal cancer.

## Description

### TECHNICAL FIELD

The present invention belongs to the field of biotechnology, and relates to a composition for detecting esophageal cancer and its use in disease detection, and in particular to a composition for detecting esophageal cancer and its corresponding kit and use.

### BACKGROUND TECHNIQUE

Esophageal cancer is a common gastrointestinal tumor. According to data from the China National Office for Cancer Prevention and Control, in 2015 the incidence of esophageal cancer in China is 478 cases per 100,000 people, and the mortality rate is 375 cases per 10,000 people, ranking fourth and third among common cancers respectively. The fatality rate of esophageal cancer is close to 80%, and it is a highly malignant cancer. Approximately 300,000 people die from esophageal cancer every year, and half of them come from China.

An important factor leading to high mortality of esophageal cancer is the low diagnosis rate of early esophageal cancer. The cure rate of early esophageal cancer is much higher than that of the cancer at middle and late stages, but due to the lack of obvious and specific symptoms of early esophageal cancer, most subjects have already developed into the middle and late stages when they are diagnosed. Clinical studies have found that it takes an average of several years for the cancer to develop from the beginning of the lesion to the clinical symptoms of the subjects; this provides an effective window period for detecting early esophageal cancer and improving the diagnosis rate of early esophageal cancer. Making full use of this window period is expected to improve the therapeutic effect of esophageal cancer and reduce the mortality of esophageal cancer.

At present, the clinical application of esophageal cancer diagnosis technology in the early detection and screening of esophageal cancer is restricted, mainly because: 1) tissue biopsy is highly invasive and not suitable for early cancer screening; 2) imaging detection technology (such as esophagography and endoscopy) is limited in terms of equipment cost, operation technology, and invasiveness etc., and it is difficult to be widely promoted as a cancer screening technology; 3) traditional serum tumor markers (such as AFP, CEA, CA125, and CA199, etc.) have low sensitivity for the detection of esophageal cancer, and cannot fully meet the requirements of early cancer screening.

### CONTENTS OF THE INVENTION

Based on this, in view of the inconvenience detection, low sensitivity, and high cost of the existing esophageal cancer detection technology, the present invention provides a composition for detecting esophageal cancer. The composition provided by the present invention may detect esophageal cancer sensitively and specifically, and the present invention also provides a kit comprising the composition and its use in detecting esophageal cancer. The kit provided by the invention has good detection sensitivity for esophageal cancer, and may detect esophageal cancer conveniently, quickly and effectively.

The invention provides a composition, a kit for in vitro detecting esophageal cancer, and uses thereof, and a method for performing detection based on the kit.

Particularly, the present invention relates to the following:
1. A composition for in vitro detecting esophageal cancer, the composition comprises:
   a nucleic acid for detecting the methylation state of a target gene,
   wherein the target gene is one or both of MT1A gene and EPO gene.
2. The composition according to item 1, wherein the target sequence of MT1A gene is shown in SEQ ID NO:1.
3. The composition according to item 1, wherein the target sequence of EPO gene is shown in SEQ ID NO:3.
4. The composition according to any one of items 1-3, wherein the nucleic acid for detecting the methylation state of a target gene comprises:
   a fragment of at least 9 nucleotides in the target sequence of the target gene,
   wherein the fragment comprises at least one CpG dinucleotide sequence.
5. The composition according to any one of items 1-4, wherein the nucleic acid for detecting the methylation state of a target gene further comprises:
   a fragment hybridizing to at least 15 nucleotides in the target sequence of the target gene under moderately stringent or stringent conditions,
   wherein the fragment comprises at least one CpG dinucleotide sequence.
6. The composition according to any one of items 1-5, which further comprises:
   a reagent for converting the 5-position unmethylated cytosine base of the target sequence of the target gene into uracil.
7. The composition according to any one of items 1-6, wherein the nucleic acid for detecting the methylation state of a target gene further comprises:
   a blocker preferentially binding to the target sequence in an unmethylated state.
8. The composition according to item 7, wherein
   the fragment of at least 9 nucleotides is the sequence of SEQ ID NO:5 and SEQ ID NO:6, or the sequence of SEQ ID NO:9 and SEQ ID NO:10,
   the fragment of at least 15 nucleotides is the sequence of SEQ ID NO:7, or the sequence of SEQ ID NO:11, and
   the blocker is the sequence of SEQ ID NO:8, or the sequence of SEQ ID NO:12.
9. An oligonucleotide for in vitro detecting esophageal cancer, which comprises:
   a fragment of at least 9 nucleotides in SEQ ID NO:1 or its complementary sequence and comprising at least one CpG dinucleotide sequence; and/or
   a fragment of at least 9 nucleotides in SEQ ID NO:3 or its complementary sequence and comprising at least one CpG dinucleotide sequence.
10. The oligonucleotide according to item 9, which further comprises:
   a fragment hybridizing to at least 15 nucleotides in SEQ ID NO:1 or its complementary sequence under moderately stringent or stringent conditions and comprising at least one CpG dinucleotide sequence; and/or
   a fragment hybridizing to at least 15 nucleotides in SEQ ID NO:3 or its complementary sequence under moderately stringent or stringent conditions and comprising at least one CpG dinucleotide sequence.
11. The oligonucleotide according to item 10, which further comprises:
   a blocker preferentially binding to the target sequence in an unmethylated state.
12. An oligonucleotide for in vitro detecting esophageal cancer, which comprises:
   the sequence of SEQ ID NO:5 and SEQ ID NO:6.
13. The oligonucleotide according to item 12, which further comprises:
   the sequence of SEQ ID NO:7.
14. The oligonucleotide according to item 13, which further comprises:
   the sequence of SEQ ID NO:8.
15. An oligonucleotide for in vitro detecting esophageal cancer, which comprises:
   the sequence of SEQ ID NO:9 and SEQ ID NO:10.
16. The oligonucleotide according to item 15, which further comprises:
   the sequence of SEQ ID NO:11.
17. The oligonucleotide according to item 16, which further comprises:
   the sequence of SEQ ID NO:12.
18. Use of MT1A gene in the preparation of a kit for in vitro detecting esophageal cancer.
19. Use of EPO gene in the preparation of a kit for in vitro detecting esophageal cancer.
20. A kit comprising the composition according to any one of items 1-8 or the oligonucleotide according to any one of items 9-17.
21. The kit according to item 20, further comprising at least one other component selected from the group consisting of:
   nucleoside triphosphate, DNA polymerase, and buffer required for the function of the DNA polymerase.
22. The kit according to item 20 or 21, further comprising: instructions.
23. Use of the composition according to any one of items 1-8 or the oligonucleotide according to any one of items 9-17 in the preparation of a kit for in vitro detecting esophageal cancer.
24. The use according to any one of items 18-19 and 23, wherein the kit for in vitro detecting esophageal cancer is used to detect esophageal cancer by a method including the following steps:
   1) separating a DNA sample comprising the target sequence of a target gene or a fragment thereof from a biological sample to be tested;
   2) determining the methylation state of the target sequence of the target gene; and
   3) judging the state of the biological sample through the detection result of the methylation state of the target sequence of the target gene, so as to achieve in vitro detecting esophageal cancer.
25. The use according to item 24, wherein the method includes the following steps:
   extracting the genomic DNA of the biological sample to be tested;
   treating the extracted genomic DNA with a reagent to convert the 5-position unmethylated cytosine base into uracil or other bases;
   contacting the DNA sample treated with the reagent with DNA polymerase and the primer of the target sequence of the target gene, and performing DNA polymerization reaction in the presence of a blocker preferentially binding to the target sequence in an unmethylated state;
   detecting an amplification product with a probe; and
   determining the methylation state of at least one CpG dinucleotide of the target sequence of the target gene based on whether the amplification product exists.
26. The use according to item 25, wherein the reagent is a bisulfite reagent.
27. A method for detecting esophageal cancer, which includes the following steps:
   separating a DNA sample comprising the target sequence of a target gene or a fragment thereof from a biological sample to be tested;
   determining the methylation state of the target sequence of the target gene; and
   judging the state of the biological sample through the detection result of the methylation state of the target sequence of the target gene, so as to achieve in vitro detecting esophageal cancer.
28. A method for detecting esophageal cancer, which includes the following steps:
   extracting the genomic DNA of a biological sample to be tested;
   treating the extracted genomic DNA with a reagent to convert the 5-position unmethylated cytosine base into uracil or other bases;
   contacting the DNA sample treated with a reagent with DNA polymerase and the primer of the target sequence of the target gene, and performing DNA polymerization reaction in the presence of a blocker preferentially binding to the target sequence in an unmethylated state;
   detecting an amplification product with a probe; and
   determining the methylation state of at least one CpG dinucleotide of the target sequence of the target gene based on whether the amplification product exists.
29. The method according to item 27 or 28, wherein
   the target gene is one or both of MT1A gene and EPO gene.
30. The method according to item 29, wherein the target sequence of MT1A gene is shown in SEQ ID NO:1.
31. The method according to item 29, wherein the target sequence of EPO gene is shown in SEQ ID NO:3.
32. The method according to item 28, wherein the reagent is a bisulfite reagent.
33. The method according to item 28, wherein the primer is:
   a fragment of at least 9 nucleotides in SEQ ID NO:1 or its complementary sequence and comprising at least one CpG dinucleotide sequence; and/or
   a fragment of at least 9 nucleotides in SEQ ID NO:3 or its complementary sequence and comprising at least one CpG dinucleotide sequence.
33. The method according to item 28, wherein the blocker is a blocker preferentially binding to the target sequence in an unmethylated state.
34. The method according to item 28, wherein the probe is:
   a fragment hybridizing to at least 15 nucleotides in SEQ ID NO:1 or its complementary sequence under moderately stringent or stringent conditions and comprising at least one CpG dinucleotide sequence; and/or
   a fragment hybridizing to at least 15 nucleotides in SEQ ID NO:3 or its complementary sequence under moderately stringent or stringent conditions and comprising at least one CpG dinucleotide sequence.
35. The method according to item 33, wherein the primer is the sequence of SEQ ID NO:5 and SEQ ID NO:6, or the sequence of SEQ ID NO:9 and SEQ ID NO:10.
36. The method according to item 33, wherein the blocker is the sequence of SEQ ID NO:8, or the sequence of SEQ ID NO:12
37. The method according to item 34, wherein the probe is the sequence of SEQ ID NO:7, or the sequence of SEQ ID NO:11.
38. A composition for in vitro detecting esophageal cancer, the composition comprises:
   a nucleic acid for detecting the methylation state of the target sequence of a target gene, and
   an antibody for detecting the concentration of a target protein,
   wherein the target gene is one or both of MT1A gene and EPO gene,
   the target protein is SNCG, i.e., γ-synuclein.
39. The composition according to item 38, wherein the target sequence of MT1A gene is shown in SEQ ID NO:1.
40. The composition according to item 38, wherein the target sequence of EPO gene is shown in SEQ ID NO:3.
41. The composition according to any one of items 38-40, wherein the nucleic acid for detecting the methylation state of the target sequence of a target gene comprises:
   a fragment of at least 9 nucleotides in the target sequence of the target gene,
   wherein the fragment comprises at least one CpG dinucleotide sequence.
42. The composition according to any one of items 38-41, wherein the nucleic acid for detecting the methylation state of a target gene further comprises:
   a fragment hybridizing to at least 15 nucleotides in the target sequence of the target gene under moderately stringent or stringent conditions,
   wherein the fragment comprises at least one CpG dinucleotide sequence.
43. The composition according to any one of items 38-42, which further comprises:
   a reagent for converting the 5-position unmethylated cytosine base of the target sequence of the target gene into uracil.
44. The composition according to any one of items 38-43, wherein the nucleic acid for detecting the methylation state of the target gene further comprises:
   a blocker preferentially binding to the target sequence in an unmethylated state.
45. The composition according to item 44, wherein
   the fragment of at least 9 nucleotides is the sequence of SEQ ID NO:5 and SEQ ID NO:6, or the sequence of SEQ ID NO:9 and SEQ ID NO:10,
   the fragment of at least 15 nucleotides is the sequence of SEQ ID NO:7, or the sequence of SEQ ID NO:11, and
   the blocker is the sequence of SEQ ID NO:8, or the sequence of SEQ ID NO:12.
46. The composition according to any one of items 38-45, which further comprises:
   a reagent for detecting the concentration of the target protein, wherein the reagent is an enzyme-linked immunosorbent assay reagent, for example, the reagent comprises an antibody-coated reaction plate, SNCG protease conjugate, substrate solution, washing solution, and stop solution for detecting the concentration of the target protein.
47. A kit comprising the composition according to any one of items 38-46 or the oligonucleotide according to any one of items 9-17.
48. The kit according to item 47, which further comprises at least one other component selected from:
   nucleoside triphosphate, DNA polymerase, and buffer required for the function of the DNA polymerase, and
   an antibody-coated reaction plate, SNCG protease conjugate, substrate solution, washing solution, and stop solution for detecting the concentration of the target protein.
49. The kit according to item 47 or 48, further comprising: instructions.
50. Use of the composition according to any one of items 38-46 or the oligonucleotide according to any one of items 9-17 in the preparation of a kit for in vitro detecting esophageal cancer.
51. The use according to any one of items 18-19 and 50, wherein the kit for in vitro detecting esophageal cancer is used to detect esophageal cancer by a method including the following steps:
   1) determining the methylation state of the target sequence of the target gene in a biological sample of a subject;
   2) determining the concentration of the target protein in the biological sample of the subject;
   3) jointly judging whether the subject has esophageal cancer through the detection result of the methylation state of the target sequence of the target gene and the concentration of the target protein, so as to achieve in vitro detecting esophageal cancer.
52. The use according to item 51, wherein the method includes the following steps:
   collecting peripheral blood of the subject, and separating plasma or serum;
   extracting free DNA in the plasma or serum;
   treating the extracted free DNA with a reagent to convert the 5-position unmethylated cytosine base into uracil or other base;
   contacting the DNA sample treated with the reagent with DNA polymerase and the primer of the target sequence of the target gene, and performing DNA polymerization reaction in the presence of a blocker preferentially binding to the target sequence in an unmethylated state;
   detecting an amplification product with a probe;
   determining the methylation state of at least one CpG dinucleotide of the target sequence of the target gene based on whether the amplification product exists; and
   determining the concentration of SNCG in the plasma or serum through immune response by using SNCG antibody.
53. The use according to item 52, wherein the reagent is a bisulfite reagent.
54. A method for detecting esophageal cancer, which includes the following steps:
   collecting a biological sample of a subject;
   determining the methylation state of the target sequence of a target gene in the biological sample of the subject;
   determining the concentration of the target protein in the biological sample of the subject; and
   jointly judging whether the subject has esophageal cancer through the detection result of the methylation state of the target sequence of the target gene and the concentration of the target protein, so as to achieve in vitro detecting esophageal cancer.
55. A method for detecting esophageal cancer, which includes the following steps:
   collecting peripheral blood of a subject, and separating plasma or serum;
   extracting free DNA from the plasma or serum;
   converting the 5-position unmethylated cytosine base into uracil or other bases;
   contacting the DNA sample treated with a reagent with DNA polymerase and the primer of the target sequence of a target gene, and performing DNA polymerization reaction in the presence of a blocker preferentially binding to the target sequence in an unmethylated state;
   detecting an amplification product with a probe;
   determining the methylation state of at least one CpG dinucleotide of the target sequence of the target gene based on whether the amplification product exists; and
   determining the concentration of SNCG in the plasma or serum through immune response by using SNCG antibody.
56. The method according to item 54 or 55, wherein
   the target gene is one or both of MT1A gene and EPO gene, and
   the target protein is SNCG.
57. The method according to item 56, wherein the target sequence of MT1A gene is shown in SEQ ID NO:1.
58. The method according to item 56, wherein the target sequence of EPO gene is shown in SEQ ID NO:3.
59. The method according to item 55, wherein the reagent is a bisulfite reagent.
60. The method according to item 55, wherein the primer is:
   a fragment of at least 9 nucleotides in SEQ ID NO:1 or its complementary sequence and comprising at least one CpG dinucleotide sequence; and/or
   a fragment of at least 9 nucleotides in SEQ ID NO:3 or its complementary sequence and comprising at least one CpG dinucleotide sequence.
61. The method according to item 55, wherein the blocker is a blocker preferentially binding to the target sequence in an unmethylated state.
62. The method according to item 55, wherein the probe is:
   a fragment hybridizing to at least 15 nucleotides in SEQ ID NO:1 or its complementary sequence under moderately stringent or stringent conditions and comprising at least one CpG dinucleotide sequence; and/or
   a fragment hybridizing to at least 15 nucleotides in SEQ ID NO:3 or its complementary sequence under moderately stringent or stringent conditions and comprising at least one CpG dinucleotide sequence.
63. The method according to item 60, wherein the primer is the sequence of SEQ ID NO:5 and SEQ ID NO:6, or the sequence of SEQ ID NO:9 and SEQ ID NO:10.
64. The method according to item 61, wherein the blocker is the sequence of SEQ ID NO:8, or the sequence of SEQ ID NO:12.
65. The method according to item 62, wherein the probe is the sequence of SEQ ID NO:7, or the sequence of SEQ ID NO:11.

The inventors of the present invention analyze the genome-wide methylation data of esophageal cancer tissues and adjacent control tissues by epigenomics and bioinformatics technology, and find two methylation genes related to esophageal cancer, and determine target sequences with abnormal methylation in these two esophageal cancer methylation genes; furthermore, the inventors of the present invention find that the methylation state of these two esophageal cancer methylation genes may be detected sensitively and specifically through the target sequences of the two genes, so as to be used to detect free DNA in peripheral blood. The detection of peripheral blood samples of esophageal cancer patients and normal control individuals shows that: the composition and detection method described in the present invention may sensitively and specifically detect esophageal cancer, including two common esophageal cancers of different cell types: squamous carcinoma and glandular carcinoma. Therefore, the present invention provides a composition and a detection method for in vitro detecting esophageal cancer, and they have important clinical application value.

Furthermore, the inventors of the present invention find that among many tumor protein markers, the joint detection of SNCG protein and the target gene methylation may significantly improve the detection of esophageal cancer. Therefore, the present invention provides a composition and a detection method for in vitro detecting esophageal cancer, and they have important clinical application value.

Other features and advantages of the present invention will be described in detail by the following specific description and claims.

### BRIEF DESCRIPTION OF THE DRAWINGS

The above and other features of the present invention will be further illustrated hereafter in conjunction with the accompanying drawings and detailed description. It should be understood that these drawings only show several exemplary embodiments according to the present invention, and therefore should not be regarded as a limitation of the protection scope of the present invention. Unless otherwise specified, the drawings are not necessarily to scale, and similar reference numerals indicate similar components.
Fig. 1 shows the results of screening the target genes of the present invention.

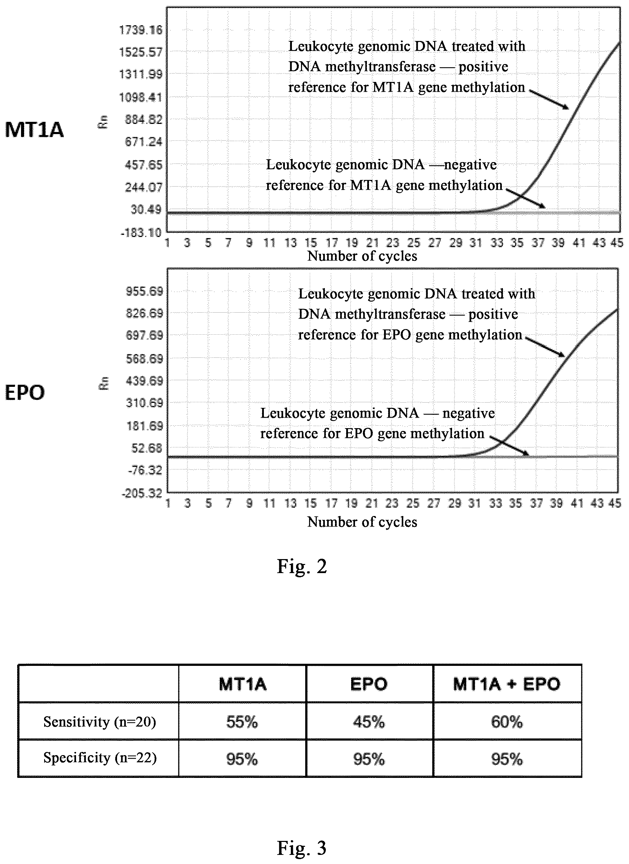

Fig. 2 shows the detection of the leukocyte genomic DNA (positive reference for the methylation state of the target sequence of the target gene) after treating the leukocyte genomic DNA (negative reference for the methylation state of the target sequence of the target gene) and DNA methyltransferase by using the composition and detection method provided by the present invention. The results show that: through using the composition and the detection method provided by the present invention, the detection result of the leukocyte genomic DNA is negative, and the detection result of the leukocyte genomic DNA treated with DNA methyltransferase is positive.
Fig. 3 shows the results of in vitro non-invasive detection of esophageal cancer through detecting the methylation state of the target sequence of the target gene by using the composition and detection method.
Fig. 4 shows the results of in vitro non-invasive detection of esophageal cancer by detecting the methylation state of the target sequence of the target gene and the concentration of the target protein by using the composition and detection method.

### DETAILED DESCRIPTION OF THE EMBODIMENTS

In one aspect, the present invention provides a composition for in vitro detecting esophageal cancer, the composition comprises a nucleic acid for detecting the methylation state of the target sequence of a target gene, and an antibody for detecting the concentration of a target protein; wherein the target gene is one or both of MT1A gene and EPO gene, and the target protein is SNCG.

The present invention provides a set of target sequences of the target genes with abnormal methylation in esophageal cancer, including the target sequences of MT1A gene and EPO gene, wherein the target sequences of MT1A gene are shown in SEQ ID NOs:1-2, and the target sequences of EPO gene are shown in SEQ ID NOs:3-4.

A target sequence of MT1A gene is shown in SEQ ID NO:1.

SEQ ID NO:1
CACCCAGGGGAGCTCAGTGGACTGTGCGCCTTGCCTTTCTGCTGCGCAAAGCCCA
GTCCAGGTCATCACCTCGGGCGGGGCGGACTCGGCTGGGCGGACTCAGCGGGGCGGGC
GCAGGCGCAGGGCGGGTCCTTTGCGTCCGGCCCTCTTTCCCCTGACCATAAAGCAGC

The complementary sequence of SEQ ID NO:1 is shown in SEQ ID NO:2.

SEQ ID NO:2
GCTGCTTTTATGGTCAGGGGAAAGAGGGCCGGACGCAAAGGACCCGCCCTGCGCC
TGCGCCCGCCCCGCTGAGTCCGCCCAGCCGAGTCCGCCCCGCCCGAGGTGATGACCTG
GACTGGGCTTTGCGCAGCAGAAAGGCAAGGCGCACAGTCCACTGAGCTCCCCTGGGTG

Preferably, a target sequence of EPO gene is shown in SEQ ID NO:3.

SEQ ID NO:3

CGCGCACGCACACATGCAGATAACAGCCCCGACCCCCGGCCAGAGCCGCAGAGTC

CCTGGGCCACCCCGGCCGCTCGCTGCGCTGCGCCGCACCGCGCTGTCCTCCCGGAGCC

GGACCGGGGCCACCGCGCCCGCTCTGCTCCGACACCGCGCCCCCTGGACAGCCGCCCT

CTCCTCCAGGCCCGTGGGGCTGGCCCTGCACCGCCGAGCTTCCCGGGATGAGGGCCCC

CGGTGTGGTCACCCGGCGCGCCCCAGGTCG

The complementary sequence of SEQ ID NO:3 is shown in SEQ ID NO:4.

CGACCTGGGGCGCGCCGGGTGACCACACCGGGGGCCCTCATCCCGGGAAGCTCGG
CGGTGCAGGGCCAGCCCACGGCCTGGAGGAGGGCGGCTGTCCAGGGGGCGCGG
TGTCGGAGCAGAGCGGGCGCGGTGGCCCCGGTCCGGCTCCGGGAGGACAGCGCGGTG
CGGCGCAGCGCAGCGAGCGGCCGGGGTGGCCCAGGGACTCTGCGGCTCTGGCCGGGG

GTCGGGGCTGTTATCTGCATGTGTGCGTGCGCG

Preferably, the nucleic acid for detecting the methylation state of a target gene comprises: a fragment of at least 9 nucleotides in the target sequence of the target gene, wherein the fragment comprises at least one CpG dinucleotide sequence. In certain preferred embodiments, for example, the DNA of the sample to be tested is converted by using bisulfite, the nucleic acid for detecting the methylation state of a target gene comprises: a fragment of at least 9 nucleotides in the target sequence of the target gene which is converted by using bisulfite, wherein the nucleotide fragment comprises at least one CpG dinucleotide sequence.

More preferably, the nucleic acid for detecting the methylation state of a target gene comprises: a fragment hybridizing to at least 15 nucleotides in the target sequence of the target gene under moderately stringent or stringent conditions, wherein the nucleotide fragment comprises at least one CpG dinucleotide sequence. In certain preferred embodiments, for example, the DNA of the sample to be tested is converted by using bisulfite, the nucleic acid for detecting the methylation state of a target gene comprises: a fragment hybridizing to at least 15 nucleotides in the target sequence of the target gene which is converted by using bisulfite under moderately stringent or stringent conditions, wherein the nucleotide fragment comprises at least one CpG dinucleotide sequence.

Preferably, the composition further comprises a reagent for converting the 5-position unmethylated cytosine base of the target sequence of the target gene into uracil. More preferably, the reagent is bisulfite.

Preferably, the nucleic acid for detecting the methylation state of a target gene further comprises a blocker preferentially binding to the DNA in an unmethylated state.

Preferably, the composition comprises one or more of the following primers, probes and/or blockers:
MT1A primer F:
   SEQ ID NO:5,
   CGGACGTAAAGGATTC
MT1A primer R:
   SEQ ID NO:6,
   GAAACGAACTCGACTAAACG
MT1A probe:
   SEQ ID NO:7,
   TGCGTTTGCGTTCGTTTCG
MT1A blocker:
   SEQ ID NO:8,
   CAAACTCAACTAAACAAACTCAACAAAACAAAC
EPO primer F:
   SEQ ID NO:9,
   AGTCGTAGAGTTTTTGGGTT
EPO primer R:
   SEQ ID NO:10,
   CAACGCGATACGACG
EPO probe:
   SEQ ID NO:11,
   CGCAACGAACGACCGA
EPO blocker:
   SEQ ID NO:12,
   GAGTTTTTGGGTTATTTTGGTTGTTTGTTG

In another aspect, the present invention provides an oligonucleotide for the in vitro detecting esophageal cancer, which comprises: a fragment of at least 9 nucleotides in SEQ ID NO:1 or its complementary sequence and comprising at least one CpG dinucleotide sequence; and/or a fragment of at least 9 nucleotides in SEQ ID NO:3 or its complementary sequence and comprising at least one CpG dinucleotide sequence.

Preferably, the oligonucleotide for in vitro detecting esophageal cancer, comprising: a fragment of at least 9 nucleotides in SEQ ID NO:1 or its complementary sequence which is converted by using bisulfite; and/or a fragment of at least 9 nucleotides in SEQ ID NO:3 or its complementary sequence which is converted by using bisulfite and comprising at least one CpG dinucleotide sequence.

The oligonucleotide for in vitro detecting esophageal cancer according to the present invention further comprises: a fragment hybridizing to at least 15 nucleotides in SEQ ID NO:1 or its complementary sequence under moderately stringent or stringent conditions and comprising at least one CpG dinucleotide sequence; and/or a fragment hybridizing to at least 15 nucleotides in SEQ ID NO:3 or its complementary sequence under moderately stringent or stringent conditions and comprising at least one CpG dinucleotide sequence.

Preferably, the oligonucleotide for in vitro detecting esophageal cancer comprises: a fragment hybridizing to at least 15 nucleotides in SEQ ID NO:1 or its complementary sequence which is converted by using bisulfite under moderately stringent or stringent conditions and comprising at least one CpG dinucleotide sequence; and/or a fragment hybridizing to at least 15 nucleotides in SEQ ID NO:3 or its complementary sequence which is converted by using bisulfite under moderately stringent or stringent conditions and comprising at least one CpG dinucleotide sequence.

The oligonucleotide for in vitro detecting esophageal cancer according to the present invention further comprises: a blocker preferentially binding to DNA in an unmethylated state.

In a particular embodiment, the oligonucleotide for in vitro detecting esophageal cancer comprises the sequences of SEQ ID NO:5 and SEQ ID NO:6. Further, it also comprises the sequence of SEQ ID NO:7. Further, it also includes the sequence of SEQ ID NO:8.

In another particular embodiment, the oligonucleotide for in vitro detecting esophageal cancer comprises the sequences of SEQ ID NO:9 and SEQ ID NO:10. Further, it also comprises the sequence of SEQ ID NO:11. Further, it also includes the sequence of SEQ ID NO:12.

In another aspect, the present invention provides a kit comprising the composition. The kit also comprises at least one other component selected from the group consisting of: nucleoside triphosphate, DNA polymerase, and buffer required for the function of the DNA polymerase.

The composition for in vitro detecting esophageal cancer according to the present invention further comprises: an antibody for detecting the concentration of a target protein, and the target protein is γ-synuclein (SNCG).

The invention also relates to use of MT1A gene, EPO gene, and SNCG protein in the preparation of a kit for in vitro detecting esophageal cancer.

Wherein, MT1A is metallothionein 1A, which is located in the q13 region of human chromosome 16 and belongs to the metallothionein gene family. Metallothionein is a small protein, which is rich in cysteine, shortage of amino acids containing aromatic groups, and is capable of binding divalent heavy metal ions. Metallothionein is an antioxidant that may protect cells from free radicals containing hydroxyl groups, maintain the balance of metal ions in cells, and at the same time play a role in removing the toxicity of heavy ions. The loss of metallothionein gene function will lead to pathological phenomena such as cancer.

EPO gene is an erythropoietin gene, which is located in the q22.1 region of human chromosome 7. The protein encoded by this gene is a glycosylated cytokine secreted by cells. When erythropoietin binds to the corresponding receptor, it may promote the synthesis of erythrocytes.

The SNCG protein is synuclein-y, which is the product of SNCG gene. SNCG gene i.e., synuclein gamma is located in the q23.2 region of human chromosome 10. This gene is a member of the synuclein gene family, and its protein sequence is as follows:
The amino acid sequence of the protein encoded by SNCG gene is:

MDVFKKGFSIAKEGVVGAVEKTKQGVTEAAEKTKEGVMYVGAKTKENVVQSVTSVAEKT
KEQANAVSEAVVSSVNTVATKTVEEAENIAVTSGVVRKEDLRPSAPQQEGEASKEKEEVAE
EAQSGGD

In another aspect, the present invention provides a method for in vitro detecting esophageal cancer, wherein the method includes the following steps:
1) separating the target sequence of a target gene or a fragment thereof from a biological sample to be tested;
2) determining the methylation state of the target sequence of the target gene; and
3) judging the state of the biological sample through the detection result of the methylation state of the target sequence of the target gene, so as to achieve in vitro detecting esophageal cancer.

According to certain preferred embodiments, the method further includes the following steps:
1) extracting the genomic DNA of the biological sample to be tested;
2) treating the DNA obtained from step 1) with a reagent to convert the 5-position unmethylated cytosine base into uracil or other bases, i.e., converting 5-position unmethylated cytosine base in the target sequence of the target gene into uracil or other bases, wherein the converted base has a hybridization performance different from the 5-position unmethylated cytosine base and is detectable;
3) contacting the DNA sample treated in step 2) with DNA polymerase and the primer of the target sequence of the target gene, so that the treated target sequence of the target gene is amplified to produce an amplification product or is not amplified; if the treated target sequence of the target gene undergoes a DNA polymerization reaction, an amplification product is produced; or if the treated target sequence of the target gene does not undergo the DNA polymerization reaction, it is not amplified;
4) detecting the amplification product with a probe; and
5) determining the methylation state of at least one CpG dinucleotide of the target sequence of the target gene based on whether the amplification product exists.

In another aspect, the present invention provides a method for in vitro detecting esophageal cancer, the method includes the following steps:
1) determining the methylation state of the target sequence of a target gene in the biological sample of a subject;
2) determining the concentration of a target protein in the biological sample of the subject; and
3) jointly judging whether the subject has esophageal cancer through the detection result of the methylation state of the target sequence of the target gene and the concentration of the target protein, so as to achieve in vitro detecting esophageal cancer.

Wherein, the biological sample is plasma or serum separated from the peripheral blood of the subject.

According to certain preferred embodiments, the method further includes the following steps:
1) collecting peripheral blood of a subject, and separating plasma or serum;
2) extracting free DNA from the plasma or serum;
3) treating the free DNA obtained in step 2) with a reagent to convert the 5-position unmethylated cytosine base into uracil or other bases, i.e., converting 5-position unmethylated cytosine base in the target sequence of a target gene into uracil or other bases, wherein the converted base has a hybridization performance different from the 5-position unmethylated cytosine base and is detectable;
4) contacting the free DNA treated in step 3) with DNA polymerase and the primer of the target sequence of the target gene, so that the treated target sequence of the target gene is amplified to produce an amplification product or is not amplified; if the treated target sequence of the target gene undergoes a DNA polymerization reaction, an amplification product is produced; or if the treated target sequence of the target gene does not undergo the DNA polymerization reaction, it is not amplified;
5) detecting the amplification product with a probe;
6) determining the methylation state of at least one CpG dinucleotide of the target sequence of the target gene based on whether the amplification product exists; and
7) determining the concentration of SNCG in the plasma or serum through immune response by using SNCG antibody.

Preferably, a typical primer comprises a fragment of the target sequence of the target gene, and the fragment of the target sequence of the target gene comprises a fragment that is respectively equivalent to, complementary to, or hybridized under moderate or stringent conditions to at least 9 nucleotides selected from SEQ ID NOs:1-2 and SEQ ID NOs:3-4.

Preferably, a typical probe is a fragment of the target sequence of the target gene, and the fragment of the target sequence of the target gene comprises a fragment that is respectively equivalent to, complementary to, or hybridized under moderate or stringent conditions to at least 15 nucleotides selected from SEQ ID NOs:1-2 and SEQ ID NOs:3-4.

Preferably, a typical blocker is a blocker preferentially binding to DNA in an unmethylated state.

Preferably, one or more of the primers, probes and/or blockers are as follows:
MT1A primer F:
   SEQ ID NO:5,
   CGGACGTAAAGGATTC
MT1A primer R:
   SEQ ID NO:6,
   GAAACGAACTCGACTAAACG
MT1Aprobe:
   SEQ ID NO:7,
   TGCGTTTGCGTTCGTTTCG
MT1A blocker:
   SEQ ID NO:8,
   CAAACTCAACTAAACAAACTCAACAAAACAAAC
EPO primer F:
   SEQ ID NO:9,
   AGTCGTAGAGTTTTTGGGTT
EPO primer R:
   SEQ ID NO:10,
   CAACGCGATACGACG
EPO probe:
   SEQ ID NO:11,
   CGCAACGAACGACCGA
EPO blocker:
   SEQ ID NO:12,
   GAGTTTTTGGGTTATTTTGGTTGTTTGTTG

In addition, the contact or amplification includes using at least one of the following methods: using a heat-resistant DNA polymerase as the amplification enzyme; using a polymerase lacking 5-3' exonuclease activity; using polymerase chain reaction (PCR); and producing amplification product nucleic acid molecules with detectable marker.

According to certain preferred embodiments, the methylation state of at least one CpG dinucleotide in the target sequence of a target gene is determined by the cycle threshold (Ct value) of the PCR reaction. By using PCR reaction to analyze DNA in a biological sample, the methylation state of the target sequence of the target gene may be easily detected, thereby quickly and conveniently judging whether the tested sample is positive according to the cycle threshold of the PCR reaction. Thus, a non-invasive and rapid in vitro detection method for esophageal cancer is provided.

The biological sample is selected from a cell line, a histological section, a tissue biopsy/a paraffin-embedded tissue, body fluids, stool, colon effluent, urine, plasma, serum, whole blood, separated blood cells, cells separated from blood, or a combination thereof. The biological sample is selected from whole blood, plasma, or serum of peripheral blood.

The present invention also provides a kit comprising the composition. Typically, the kit comprises a container for holding a biological sample of a subject. In addition, the kit also comprises instructions for using and interpreting a detection result.

The invention provides a method for non-invasively in vitro detecting esophageal cancer through detecting the methylation state of the target sequence of a target gene and the concentration of a target protein. The inventors find that there is a significant difference between the methylation state of the target sequence of MT1A gene and EPO gene in esophageal cancer tissue and the methylation state of the target sequence of the target genes in normal esophageal tissue: the target sequence of MT1A gene and EPO gene in esophageal cancer tissue is methylated, while the target sequence of MT1A gene and EPO gene in normal esophageal tissue is not methylated. Therefore, this application provides a method for in vitro detecting esophageal cancer through detecting the methylation state of the target sequence of MT1A gene and EPO gene in a sample. The method provided by the present invention may detect esophageal cancer non-invasively and quickly.

The inventors further find that, compared with detecting the methylation state of the target sequence of the target gene alone, jointly detecting the methylation state of the target sequence of the target gene and the concentration of the target protein may significantly improve the detection rate of esophageal cancer, while has no significant effect on the specificity of the detection. Therefore, this application provides a method for in vitro detecting esophageal cancer through detecting the methylation state of the target sequences of MT1A gene and EPO gene and the concentration of SNCG protein in a sample. The method provided by the present invention may detect esophageal cancer non-invasively and quickly.

Unless otherwise defined, relevant technical and scientific terms in this specification have the same meanings as commonly understood by those skilled in the art. Although methods and materials similar or identical to those described herein may be used in experiments or practical applications, the materials and methods are described below. In case of conflict, the specification, including the definitions therein, shall prevail. In addition, the materials, methods and examples are for illustrative purposes only and not restrictive.

The invention also provides a composition capable of sensitively and specifically detecting the methylation state of the target sequence of a target gene and the concentration of a target protein; and a method and kit for non-invasively detecting esophageal cancer in vitro.

The examples of the composition, kit, nucleic acid sequence, and detection method according to the present invention are described below. The first group of embodiments disclose a target gene and the target sequence of the target gene; the second group of embodiments disclose a composition for detecting the methylation state of the target sequence of a target gene, including a nucleic acid for detecting the methylation state of the target sequence of a target gene; the third group of embodiments disclose a method for in vitro non-invasive detection of esophageal cancer by detecting the methylation state of the target sequence of a target gene and the concentration of a target protein.

Preferably, the sequence of the nucleic acid detection comprises a fragment of at least 9 nucleotides in the target sequence of the target gene, wherein the fragment of nucleotides comprises at least one CpG dinucleotide sequence; in certain preferred embodiments, for example, the DNA of a sample to be tested is converted by using bisulfite, and the sequence of the nucleic acid detection comprises a fragment of at least 9 nucleotides in the target sequence of the target gene which is converted by using bisulfite, wherein the fragment of nucleotides comprises at least one CpG dinucleotide sequence;

More preferably, the nucleic acid detection sequence respectively comprises a fragment hybridizing to at least 15 nucleotides in the target sequence of the target gene under moderately stringent or stringent conditions, wherein the fragment of nucleotides comprises at least one CpG dinucleotide sequence; in certain preferred embodiments, for example, the DNA of a sample to be tested is converted by using bisulfite, and the sequence of the nucleic acid detection comprises a fragment hybridizing to at least 15 nucleotides in the target sequence of the target gene which is converted by using bisulfite under moderately stringent or stringent conditions, wherein the fragment of nucleotides comprises at least one CpG dinucleotide sequence

In certain embodiments, the composition further compries a reagent for converting the 5-position unmethylated cytosine base of the target sequence of the target gene into uracil. Preferably, the reagent is bisulfite. Bisulfite modification of DNA is a known tool for assessing the methylation state of CpG. In the DNA of eukaryotic cells, 5-methylcytosine is the most common covalent base modification. 5-methylcytosine cannot be identified by sequencing, because 5-methylcytosine and cytosine have the same base pairing behavior. In addition, during the PCR amplification process, the epigenetic information carried by 5-methylcytosine is completely lost. The most commonly used method to analyze the presence of 5-methylcytosine in DNA is based on the specific reaction between bisulfite and cytosine; after the subsequent alkaline hydrolysis, the unmethylated cytosine is converted into uracil corresponding to thymine in pairing behavior; but 5-methylcytosine remains unmodified under these conditions. As a result, the original DNA is converted in this way, so that 5-methylcytosine, which was originally indistinguishable from cytosine in its hybridization behavior, may now be detected as the only remaining cytosine by conventional known molecular biology techniques, for example, by amplification and hybridization. All these technologies are based on different base pairing properties, and may now be fully utilized. Therefore, this application typically provides the joint use of bisulfite technology with one or more methylation assays to determine the methylation state of CpG dinucleotide sequences in the target sequence of a target gene. In addition, the method according to the present invention is suitable for analyzing a heterogeneous biological sample, such as tumor cells in blood or stool with low concentration. Therefore, when analyzing the methylation state of the CpG dinucleotide sequence in this sample, those skilled in the art may use a quantitative assay to determine the methylation level of a specific CpG dinucleotide sequence (e.g., percentage, number of copies, ratio, proportion, or extent), not the methylation state. Correspondingly, the term methylation status or methylation state should also be considered to refer to a value reflecting the methylation state of the CpG dinucleotide sequence.

In certain embodiments, the method according to the present application specifically includes: 1) extracting the genomic DNA of the biological sample to be tested; 2) treating the DNA obtained from step 1) with a reagent to convert the 5-position unmethylated cytosine base into uracil or other bases, i.e., converting 5-position unmethylated cytosine base in the target sequence of a target gene into uracil or other bases, wherein the converted base has a hybridization performance different from the 5-position unmethylated cytosine base and is detectable; 3) contacting the DNA sample treated in step 2) with DNA polymerase and the primer of the target sequence of the target gene, so that the treated target sequence of the target gene is amplified to produce an amplification product or is not amplified; if the treated target sequence of the target gene undergoes a DNA polymerization reaction, an amplification product is produced; or if the treated target sequence of the target gene does not undergo the DNA polymerization reaction, it is not amplified; 4) detecting the amplification product with a probe; and 5) determining the methylation state of at least one CpG dinucleotide of the target sequence of the target gene based on whether the amplification product exists.

In certain embodiments, the method according to the present application specifically includes: 1) collecting peripheral blood of a subject; 2) extracting free DNA from plasma or serum; 3) treating the free DNA obtained in step 2) with a reagent to convert the 5-position unmethylated cytosine base into uracil or other bases, i.e., converting 5-position unmethylated cytosine base in the target sequence of the target gene into uracil or other bases, wherein the converted base has a hybridization performance different from the 5-position unmethylated cytosine base and is detectable; 4) contacting the DNA sample treated in step 3) with DNA polymerase and the primer of the target sequence of the target gene, so that the treated target sequence of the target gene is amplified to produce an amplification product or is not amplified; if the treated target sequence of the target gene undergoes a DNA polymerization reaction, an amplification product is produced; or if the treated target sequence of the target gene does not undergo the DNA polymerization reaction, it is not amplified; 5) detecting the amplification product with a probe; 6) determining the methylation state of at least one CpG dinucleotide of the target sequence of the target gene based on whether the amplification product exists; and 7) determining the concentration of the target protein in the plasma or serum through immune response (preferably, enzyme-linked immunosorbent assay, ELISA) by using SNCG antibody.

Typically, the contacting or amplification includes using at least one of the following methods: using a heat-resistant DNA polymerase as the amplification enzyme; using a polymerase lacking 5-3' exonuclease activity; using PCR; producing a nucleic acid molecule of the amplification product with a detectable label. Preferably, PCR is used to determine the methylation state, such as "fluorescence-based real-time PCR technology", methylation-sensitive single nucleotide primer extension reaction (Ms-SNuPE), methylation specific PCR (MSP), and methylated CpG island amplification (MCA) and other measurement methods are used to determine the methylation state of at least one CpG dinucleotide of the target sequence of a target gene. Wherein, the "fluorescence-based real-time PCR" assay is a high-throughput quantitative methylation assay, which uses fluorescence-based real-time PCR (TaqMan) technology, and no further operations are required after the PCR step. In short, the "fluorescence-based real-time PCR" method is started from a mixed sample of genomic DNA that is converted into a mixed pool of methylation-dependent sequence differences in a sodium bisulfite reaction according to standard procedures. Fluorescence-based PCR is then performed in a "biased" reaction (using PCR primers overlapping known CpG dinucleotides). Sequence differences may be produced at the level of amplification, as well as at the level of fluorescence detection amplification. The "fluorescence-based real-time PCR" assay may be used as a quantitative test of the methylation state in a genomic DNA sample, wherein sequence discrimination occurs at the level of probe hybridization. In this quantitative method, in the presence of fluorescent probes overlapping specific CpG dinucleotides, the PCR reaction provides methylation-specific amplification. A no-offset control for the starting DNA amount is provided by a reaction in which neither primer nor probe covers any CpG dinucleotides. The "fluorescence-based real-time PCR" method may be used together with any suitable probe, such as "TaqMan", "Lightcycler", etc. The TaqMan probe is double-labeled with a fluorescent reporter and a quencher, and is designed to be specific to a relatively high GC content region, so that it is melted at a temperature of approximately 10°C higher than that of the forward or reverse primer in the PCR cycle. This allows the TaqMan probe to remain fully hybridized during the PCR annealing/extension step. When Taq polymerase enzymatically synthesizes a new strand in PCR, it will eventually encounter an annealed TaqMan probe. The 5' to 3' endonuclease activity of Taq polymerase will then replace it by digesting the TaqMan probe, thereby releasing the fluorescent reporter molecule for quantitative detection of its current unquenched signal by using a real-time fluorescence detection system. Typical reagents for "fluorescence-based real-time PCR" assay may include, but are not limited to: PCR primers for the target sequence of a target gene; non-specific amplification blockers; TaqMan or Lightcycler probes; optimized PCR buffers and deoxynucleotides; and Taq polymerase, etc.

Wherein, the determination of concentration of SNCG in plasma or serum through immune response by using SNCG antibody is to detect the expression level of SNCG protein in serum by enzyme-linked immunosorbent assay (ELISA).

Preferably, the ELISA is a "sandwich method", and particularly the method includes the following steps:
1) using SNCG monoclonal antibody (mouse) to coat the 96-well plate;
2) adding 10-fold diluted clinical serum sample or plasma sample, and the serially diluted human SNCG protein solution;
3) adding horseradish peroxidase (HRP) labeled mouse anti-human SNCG polyclonal antibody (IgG) to each well;
4) incubating the immunoreaction plate containing the above mixture at room temperature and under shaking condition, then washing each reaction well with washing solution, followed by adding a chromogenic substrate;
5) adding stop solution to terminate the color reaction, followed by using a plate reader to detect the spectrum of each reaction well;
6) establishing a standard curve based on the detection values of serially diluted human SNCG protein solutions, and quantifying the clinical serum samples based on the standard curve.

In a specific embodiment of the present invention, the critical values of esophageal cancer samples and normal samples relative to the protein levels of SNCG gene in serum or plasma are determined, based on the protein levels of the SNCG gene in the serum or plasma of a certain number of esophageal cancer samples and normal samples.

### EXAMPLE

### Example 1

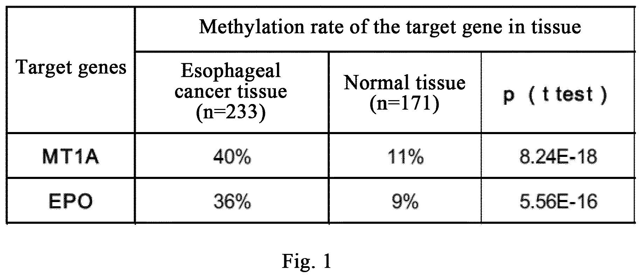

By analyzing the whole genome methylation chip (HumanMethylation450k chip from Illumina) data from 233 cases of esophageal cancer tissues and 171 cases of normal esophageal tissues, the inventors find that the methylation levels of MT1A gene and EPO gene in esophageal cancer tissue are significantly higher than those in normal esophagus tissue (analysis results are shown in Fig. 1). Furthermore, the inventors analyze the probe sequences of MT1A gene and EPO gene on the whole genome methylation chip and the corresponding methylation rate data, and find the sequence fragments with the most obvious methylation discrimination in these two target genes in the esophageal cancer tissue and normal esophageal tissue, and they are determined as the target sequences of the two target genes.

The target sequence of MT1A gene is shown in SEQ ID NO:1.

SEQ ID NO:1
CACCCAGGGGAGCTCAGTGGACTGTGCGCCTTGCCTTTCTGCTGCGCAAAGCCCA
GTCCAGGTCATCACCTCGGGCGGGGCGGACTCGGCTGGGCGGACTCAGCGGGGCGGGC
GCAGGCGCAGGGCGGGTCCTTTGCGTCCGGCCCTCTTTCCCCTGACCATAAAGCAGC

The complementary sequence of the target sequence of MT1A gene is shown in SEQ ID NO:2.

SEQ ID NO:2
GCTGCTTTATGGTCAGGGGAAAGAGGGCCGGACGCAAAGGACCCGCCCTGCGCC
TGCGCCCGCCCCGCTGAGTCCGCCCAGCCGAGTCCGCCCCGCCCGAGGTGATGACCTG
GACTGGGCTTTGCGCAGCAGAAAGGCAAGGCGCACAGTCCACTGAGCTCCCCTGGGTG

The target sequence of EPO gene is shown in SEQ ID NO:3.

SEQ ID NO:3

CGCGCACGCACACATGCAGATAACAGCCCCGACCCCGGCCAGAGCCGCAGAGTC

CCTGGGCCACCCCGGCCGCTCGCTGCGCTGCGCCGCACCGCGCTGTCCTCCCGGAGCC

GGACCGGGGCCACCGCGCCCGCTCTGCTCCGACACCGCGCCCCCTGGACAGCCGCCCT

CTCCTCCAGGCCCGTGGGGCTGGCCCTGCACCGCCGAGCTTCCCGGGATGAGGGCCCC

CGGTGTGGTCACCCGGCGCGCCCCAGGTCG

The complementary sequence of the target sequence of EPO gene is shown in SEQ ID NO:4.

SEQ ID NO:4

CGACCTGGGGCGCGCCGGGTGACCACACCGGGGGCCCTCATCCCGGGAAGCTCGG

CGGTGCAGGGCCAGCCCCACGGGCCTGGAGGAGAGGGCGGCTGTCCAGGGGGCGCGG

TGTCGGAGCAGAGCGGGCGCGGTGGCCCCGGTCCGGCTCCGGGAGGACAGCGCGGTG

CGGCGCAGCGCAGCGAGCGGCCGGGGTGGCCCAGGGACTCTGCGGCTCTGGCCGGGG

GTCGGGGCTGTTATCTGCATGTGTGCGTGCGCG

### Example 2

Step 1: obtaining the DNA of the biological sample to be analyzed. The source may be any suitable source, such as cell lines, histological sections, biopsy tissues, paraffin-embedded tissues, body fluid, stool, urine, plasma, serum, whole blood, separated blood cells, cells separated from blood, and all possible combinations thereof. The DNA is then separated from the sample by any standard means in the prior art. In short, when DNA is encapsulated in the cell membrane, the biological sample must be broken and lysed by enzymatic, chemical or mechanical means. The protein and other contaminants are then removed, for example by digestion with protein kinase K. Then the DNA is recovered from the solution. This may be achieved by various methods, including salting out, organic extraction, or binding of DNA to a solid support. The choice of a method will be affected by many factors, including time, cost, and the amount of DNA required. When the sample DNA is not encapsulated in the cell membrane (for example, circulating DNA from a blood sample), standard methods for separating and/or purifying DNA in the prior art may be used. These methods include the use of protein degradation reagents, e.g., chaotropic salts, such as guanidine hydrochloride or urea; or detergents, such as sodium dodecyl sulfonate (SDS), cyanogen bromide. Other methods include but are not limited to ethanol precipitation or propanol precipitation, vacuum concentration by centrifugation, and the like. Those skilled in the art may also use devices, e.g., such as filters for ultrafiltration, silicon surfaces or membranes, magnetic particles, polystyrene particles, polystyrene surfaces, positively charged surfaces and positively charged membranes, charged membranes, charged surface, charged conversion film, and charged conversion surface. Once the nucleic acid is extracted, the DNA is used for analysis.

In this embodiment, the biological sample DNA is leukocyte genomic DNA, and leukocyte genomic DNA treated with DNA methyltransferase. The target sequence of the target gene of leukocyte genomic DNA is in an unmethylated state, so leukocyte genomic DNA is a negative reference for the methylation state of the target sequence of the target gene. The target sequence of the target gene of the leukocyte genomic DNA treated with DNA methyltransferase is in the methylation state, so the leukocyte genomic DNA treated with DNA methyltransferase is a positive reference for the methylation state of the target sequence of the target gene.

Step 2: treating the above two DNA samples separately so that the 5-position unmethylated cytosine base is converted into uracil, thymine, or another base not used for cytosine in hybridization behavior. Preferably, this is achieved by treating with a bisulfite reagent. The term "bisulfite reagents" refers to reagents that include bisulfite, acid sulfite, or combinations thereof, as disclosed herein they may be used to distinguish a methylated CpG dinucleotide sequence from an unmethylated CpG dinucleotide sequence. Preferably, the bisulfite treatment is performed in the presence of a denaturing solvent, such as but not limited to n-alkyl glycol, especially diethylene glycol dimethyl ether (DME), or is performed in the presence of dioxane or dioxane derivatives. In a preferred embodiment, the denaturing solvent is used at a concentration of 1% to 35% (v/v). It is also preferred that the bisulfite reaction is carried out in the presence of a scavenger, for example, but not limited to chromane derivatives, such as 6-hydroxy-2,5,7,8,-tetramethylchromane 2-carboxylic acid, or trihydroxybenzoic acid and its derivatives, such as gallic acid. This bisulfite conversion is preferably carried out at a reaction temperature of 30-70°C, wherein the temperature increases to over 85°C for a short time during the reaction. The DNA treated with bisulfite is preferably purified before quantification. This may be achieved by any method known in the prior art, such as but not limited to ultrafiltration.

The third step: amplifying the treated DNA fragments by using the primers and the amplification enzymes according to the present invention. Several DNA fragments may be amplified simultaneously in the same reaction vessel. Preferably, the length of the amplification product is 100-2,000 base pairs. When the genomic DNA of the biological sample to be tested is a mixture of methylated and unmethylated states, especially when DNA in methylated state is far less than DNA in unmethylated state, for example, free DNA in the peripheral blood of a subject with cancer, in order to improve the amplification specificity of PCR amplification primers, the present invention uses a blocker specific to the target sequence of the target gene in the PCR reaction system. The 5' end of the nucleotide sequence of the blocker and the 3' end of the nucleotide sequence of the forward (F) or reverse (R) primer have an overlap area greater than or equal to 5 nucleotides; the blocker and the forward (F) or reverse (R) primer are complementary to the same strand of the target sequence DNA of the target gene; the melting temperature of the blocker is 5°C or more higher than that of the forward (F) or reverse (R) primer; the nucleotide sequence of the blocker comprises at least one CpG dinucleotide sequence, and is complementary to the sequence of the target sequence DNA of the target gene that does not undergo methylation after the bisulfite conversion. Therefore, when the genomic DNA of the biological sample to be tested is a mixture of methylated and unmethylated states, especially when DNA in a methylated state is far less than that in an unmethylated state, the unmethylated DNA which is converted by bisulfite will preferentially bind to the blocker, thereby inhibiting the binding of the DNA template to the PCR primers, so that PCR amplification does not occur; while the DNA in methylated state does not bind to the blocker, and therefore binds to the primers, and PCR amplification occurs. After that, the fragments obtained by amplification are directly or indirectly detected. It is preferred that the label is in the form of a fluorescent label, a radionuclide, or an attachable molecular fragment.

According to the target sequences of the target genes SEQ ID NOs:1-2 and SEQ ID NOs:3-4, the sequences of primers, probes and blockers (SEQ ID NOs:5-12) for detecting the methylation state of the target sequences of the two target genes (MT1A and EPO) are designed in the present invention:
Preferably, one or more of the primers, probes and/or blockers are as follows:
MT1A primer F:
   SEQ ID NO:5,
   CGGACGTAAAGGATTC
MT1A primer R:
   SEQ ID NO:6,
   CGGACGTAAAGGATTC
MT1A probe:
   SEQ ID NO:7,
   TGCGTTTGCGTTCGTTTCG
MT1A blocker:
   SEQ ID NO:8,
   CAAACTCAACTAAACAAACTCAACAAAACAAAC
EPO primer F:
   SEQ ID NO:9,
   AGTCGTAGAGTTTTTGGGTT
EPO primer R:
   SEQ ID NO:10,
   CAACGCGATACGACG
EPO probe:
   SEQ ID NO:11,
   CGCAACGAACGACCGA
EPO blocker:
   SEQ ID NO:12,
   GAGTTTTTGGGTTATTTTGGTTGTTTGTTG

In the present invention, various commercial real-time PCR equipment may be used to perform real-time PCR assay according to the standard operations of the prior art. According to certain particular embodiments, the real-time PCR assay is performed on Life Technologies instrument (7500 Fast). The PCR reaction mixture consists of 25-40ng of DNA template converted by bisulfite, and 300-600nM primer and blocker, 150-300nM probe, 1UTaq polymerase, 50-400µM each dNTP, 1-10mM MgCl₂, and 2XPCR buffer to a final volume of 2-100 µl. At 85-99°C for 3-60 minutes to amplify the sample with a pre-cycle, followed by 35-55 cycles of annealing at 50-72°C for 1-30 seconds, and annealing and extending at 45-80°C for 5-90 seconds, and then denaturing at 85-99°C for 5-90 seconds. By observing the amplification only on the methylated target sequence of the target gene, the gene fragment is detected with a probe specific to the CpG island region of the target sequence of the target gene containing 5-methylcytosine. Furthermore, in certain particular embodiments, β-actin gene (ACTB) may be used as an internal reference for PCR, and β-actin gene amplicons may be created by using primers complementary to the sequence of β-actin gene, and a specific probe is used to detect the amplicons of the β-actin gene. Each sample is subjected to at least one real-time PCR, and in certain embodiments, two or three times of real-time PCR assays are performed.

The experimental results are shown in Fig. 2: the detection of leukocyte genomic DNA (a negative reference for the methylation state of the target sequence of the target gene) by the composition and detection method according to the present invention indicates that PCR amplification does not occur, and the detection result is negative; i.e., the target sequence of the target gene of the tested DNA sample is not methylated; while the detection of leukocyte genomic DNA treated with DNA methyltransferase (a positive reference for the methylation state of the target sequence of the target gene) by the composition and detection method according to the present invention indicates that PCR amplification occurs, and the detection result is positive; i.e., the target sequence of the target gene of the tested DNA sample is methylated. It may be judged from this, the composition and detection method according to the present invention may specifically detect the methylation state of the target sequence of a target gene.

### Example 3

According to a specific embodiment of the present application, the Ct value (i.e., the critical value) capable of effectively distinguishing esophageal cancer gene from normal target gene is determined based on the average Ct values of the detection results of a certain number of esophageal cancer samples and normal samples. The methylation state of at least one CpG dinucleotide of the target sequence of the target gene is determined by the cycle threshold (Ct value) of the polymerase chain reaction. Whether the analysis result on the target gene is negative (normal) or positive (esophageal cancer) is determined by comparing the Ct values of the tested samples with a preset threshold.

This example includes the following steps:
First, plasma samples from 20 esophageal cancer patients and 22 normal people are collected. All samples come from Biochain Company. Then free DNA of the peripheral blood is extracted from a test sample, and the DNA sample is pre-treated so that the 5-position unmethylated cytosine base is converted into uracil, thymine or another base not used for cytosine in hybridization behavior. In this example, the pretreatment is achieved by treating with a bisulfite reagent. The DNA extraction and the treatment may be carried out by using any standard means in the prior art. Particularly, in this example, the extraction of DNA of all samples and the bisulfite modification of the DNA are performed by using the plasma processing kit from Biochain Company.

Then, a combination of the above-mentioned target gene primers, probes and blocker is added to the above-mentioned processed DNA samples of 20 esophageal cancer patients and 22 normal people, and the methylation state of the target sequence of the target gene is detected by PCR, wherein the PCR in this experimental example is performed on Life Technologies instrument (7500). The PCR reaction mixture consists of 35ng of DNA template converted by bisulfite, and 450nM primer and blocker, 225nM probe, 1UTaq polymerase, 200µM of each dNTP, 4.5mM MgCl₂, and 2XPCR buffer to a final volume of 50µl. At 94°C for 20 minutes to amplify the sample with a pre-cycle, followed by 45 cycles of annealing at 62°C for 5 seconds, and annealing and extending at 55.5°C for 35 seconds, and then denaturing at 93°C for 30 seconds.

Finally, Ct values of the target sequence of the target gene in the real-time PCR for DNA samples from 20 esophageal cancer patients and 22 normal people are respectively detected. The detection results are shown in Fig. 3: 1) selecting a specific critical value, preferably the critical value Ct=37, and a patient with esophageal cancer may be effectively detected through the detection of the methylation state of the target sequence of the target gene by using the composition and detection method according to the present invention; in this example, when the Ct value as the critical value is 37, the sensitivity of detecting esophageal cancer by methylation assay of the target sequence of a target gene is 55% (MT1A) and 45% (EPO) respectively; 2) the methylation of the target sequence of a target gene has good specificity, and the detection of normal people shows that the specificity of normal people detection for methylation of the target sequences of the target genes are respectively 95% (MT1A) and 95% (EPO); 3) with a combination of the results of MT1A gene and EPO gene methylation assay, the sensitivity of detecting esophageal cancer may be increased to 60%, while the specificity is unchanged, still 95%.

### Example 4

Afterwards, with respect to the additional plasma samples from 63 patients with esophageal cancer, the free DNA in the plasma is then extracted, and the genomic DNA sample is pretreated so that the 5'-position unmethylated cytosine base is converted into uracil, thymine, or another base not used for cytosine in hybridization behavior. In this example, the pretreatment is achieved by treating with a bisulfite reagent. The DNA extraction and the treatment may be carried out by using any standard means in the prior art. Particularly, in this example, the extraction of DNA of all samples and the bisulfite modification of the DNA are performed by using the plasma processing kit from Biochain Company. Then, a combination of the above-mentioned MT1A and EPO gene primers and probes, and a combination of the internal reference gene ACTB gene primer and probe are respectively added to the above-mentioned processed DNA samples of 63 esophageal cancer subjects, and the methylation states of the target sequences of MT1A and EPO gene are detected by PCR, wherein the PCR amplification condition adopted in this experimental example is: performing the real-time PCR on Life Technologies instrument (7500). The PCR reaction mixture consists of 35ng of DNA template converted by bisulfite, and 450nM primer, 225nM probe, 1UTaq polymerase, 200µM of each dNTP, 4.5mM MgCl₂, and 2XPCR buffer to a final volume of 30µl. At 94°C for 20 minutes to amplify the sample with a pre-cycle, followed by 45 cycles of annealing at 62°C for 5 seconds, and annealing and extending at 55.5°C for 35 seconds, and then denaturing at 93°C for 30 seconds.

At the same time, the plasma samples of these 63 esophageal cancer subjects are tested for the serum protein levels of SNCG gene. The serum protein level of SNCG gene is detected by enzyme-linked immunosorbent assay (ELISA). This ELISA detection technology uses the "sandwich method" to detect the serum protein level of the SNCG gene: firstly, using SNCG monoclonal antibody (mouse) (from Biochain Company) to coat the 96-well plate (1µg/well); and adding 10-fold diluted clinical serum sample and serially diluted (2.5ng/mL-0.04ng/mL) human SNCG protein solution (50µl/well); then adding 50µl of 0.47µg/mL horseradish peroxidase (HRP) labeled mouse anti-human SNCG polyclonal antibody (IgG) (from Biochain Company) to each well. The immunoreaction plate containing the above mixture is incubated at room temperature for 3 hours under shaking condition, then washing each reaction well with washing solution, followed by adding the chromogenic substrate. After terminating the color reaction by adding 2M sulfuric acid, using a plate reader (such as Bio-RAD 550) to detect each reaction well at 450nm spectral band. Finally, a standard curve is established on the basis of the detection values of serially diluted human SNCG protein solutions, and quantifying the clinical serum samples based on the standard curve. The critical value of serum level of SNCG gene in esophageal cancer and normal individuals is 2.0ng/mL.

Finally, the levels of commonly used cancer markers (including CEA, CA199, CA125, and AFP) in 63 samples of esophageal cancer subjects are detected by using Roche's electrochemiluminescence immunoassay system.

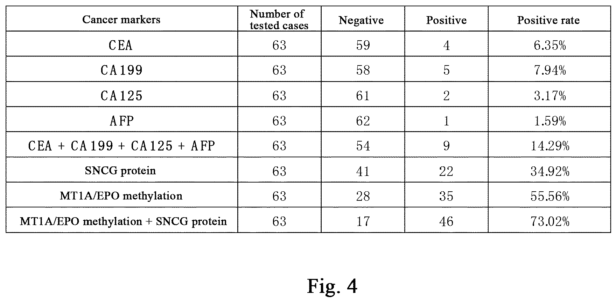

The detection results show (Fig. 4): The positive rates of the methylation detection for MT1A gene and EPO gene and SNCG protein detection for esophageal cancer in subjects with esophageal cancer are significantly higher than those of commonly used cancer markers (such as CEA, CA199, CA125, and AFP). At the same time, the joint detection of the DNA methylation of MT1A and EPO genes and SNCG protein has outstanding complementarity in the detection of esophageal cancer, and the joint detection of the two greatly improves the detection rate of esophageal cancer. In addition, the complementarity between DNA methylation detection of MT1A and EPO genes and SNCG protein detection is unique, and the commonly used cancer markers (such as CEA, CA199, CA125, and AFP) do not have this property.

The above experimental results show that the joint detection of the methylation state of the target sequences of the target genes and the concentration of the target protein may detect esophageal cancer sensitively and specifically. With the detection of the methylation DNA in target sequence of the target gene according to the present invention, in vitro non-invasive detection of esophageal cancer may be achieved, and the detection rate of esophageal cancer may also be improved.

In summary, through detecting the methylation state of the target sequences of the target genes and the concentration of the target protein, the present application utilizes the above-mentioned composition, nucleic acid sequences, kit and use thereof, and the above-mentioned detection method for in vitro detection of esophageal cancer by using the methylation state of the target sequence of the target gene and the concentration of the target protein, thereby effectively improving the sensitivity and specificity of in vitro detection of esophageal cancer. By using real-time PCR to analyze free DNA in plasma samples and using ELISA assay to analyze the concentration of the target protein in plasma, it is convenient to realize the joint detection of the methylation state of the target sequences of the target genes and the concentration of the target protein, and thereby quickly and conveniently determine whether the sample is positive based on the CT value of real-time PCR and detection concentration of ELISA assay, providing a non-invasive and convenient in vitro detection method for esophageal cancer.

Although various aspects and examples of the present invention are disclosed herein, other aspects and examples are obvious to those skilled in the art. The various aspects and examples disclosed herein are for illustrative purposes only, not for limiting purposes. The protection scope and spirit of the present invention are only determined by the appended claims.

## Claims

1. A composition for in vitro detecting esophageal cancer, the composition comprises:
a nucleic acid for detecting the methylation state of a target gene,
wherein the target gene is one or both of MT1A gene and EPO gene.

2. The composition for in vitro detecting esophageal cancer according to claim 1, the composition further comprises:
an antibody for detecting the concentration of a target protein,
wherein the target protein is SNCG, i.e., γ-synuclein.

3. The composition according to claim 1 or 2, wherein the target sequence of MT1A gene is shown in SEQ ID NO:1.

4. The composition according to claim 1 or 2, wherein the target sequence of EPO gene is shown in SEQ ID NO:3.

5. The composition according to any one of claims 1-4, wherein the nucleic acid for detecting the methylation state of a target gene comprises:
a fragment of at least 9 nucleotides in the target sequence of the target gene,
wherein the fragment comprises at least one CpG dinucleotide sequence.

6. The composition according to any one of claims 1-5, wherein the nucleic acid for detecting the methylation state of a target gene comprises:
a fragment hybridizing to at least 15 nucleotides in the target sequence of the target gene under moderately stringent or stringent conditions,
wherein the fragment comprises at least one CpG dinucleotide sequence.

7. The composition according to any one of claims 1-6, further comprising:
a reagent for converting the 5-position unmethylated cytosine base of the target sequence of the target gene into uracil.

8. The composition according to any one of claims 1-7, wherein the nucleic acid for detecting the methylation state of a target gene further comprises:
a blocker preferentially binding to the target sequence in an unmethylated state.

9. The composition according to claim 8, wherein
the fragment of at least 9 nucleotides is the sequence of SEQ ID NO:5 and SEQ ID NO:6, or the sequence of SEQ ID NO:9 and SEQ ID NO:10,
the fragment of at least 15 nucleotides is the sequence of SEQ ID NO:7, or the sequence of SEQ ID NO:11, and
the blocker is the sequence of SEQ ID NO:8, or the sequence of SEQ ID NO:12.

10. The composition according to any one of claims 2-9, the composition further comprises:
a reagent for detecting the concentration of the target protein, wherein the reagent is an enzyme-linked immunosorbent assay reagent, for example, the reagent comprises an antibody-coated reaction plate, SNCG protease conjugate, substrate solution, washing solution, and stop solution for detecting the concentration of the target protein.

11. An oligonucleotide for in vitro detecting esophageal cancer, comprising:
a fragment of at least 9 nucleotides in SEQ ID NO:1 or its complementary sequence and comprising at least one CpG dinucleotide sequence; and/or
a fragment of at least 9 nucleotides in SEQ ID NO:3 or its complementary sequence and comprising at least one CpG dinucleotide sequence.

12. The oligonucleotide according to claim 11, further comprising:
a fragment hybridizing to at least 15 nucleotides in SEQ ID NO:1 or its complementary sequence under moderately stringent or stringent conditions and comprising at least one CpG dinucleotide sequence; and/or
a fragment hybridizing to at least 15 nucleotides in SEQ ID NO:3 or its complementary sequence under moderately stringent or stringent conditions and comprising at least one CpG dinucleotide sequence.

13. The oligonucleotide according to claim 12, further comprising: a blocker preferentially binding to the target sequence in an unmethylated state.

14. An oligonucleotide for in vitro detecting esophageal cancer, comprising: the sequence of SEQ ID NO:5 and SEQ ID NO:6.

15. The oligonucleotide according to claim 14, further comprising: the sequence of SEQ ID NO:7.

16. The oligonucleotide according to claim 15, further comprising: the sequence of SEQ ID NO:8.

17. An oligonucleotide for in vitro detecting esophageal cancer, comprising: the sequence of SEQ ID NO:9 and SEQ ID NO:10.

18. The oligonucleotide according to claim 17, further comprising: the sequence of SEQ ID NO:11.

19. The oligonucleotide according to claim 18, further comprising: the sequence of SEQ ID NO:12.

20. Use of MT1A gene in the preparation of a kit for in vitro detecting esophageal cancer.

21. Use of EPO gene in the preparation of a kit for in vitro detecting esophageal cancer.

22. A kit comprising the composition according to any one of claims 1-10 or the oligonucleotide according to any one of claims 11-19.

23. The kit according to claim 22, further comprising at least one other component selected from the group consisting of:
nucleoside triphosphate, DNA polymerase, and buffer required for the function of the DNA polymerase.

24. The kit according to claim 23, further comprising at least one other component selected from the group consisting of:
an antibody-coated reaction plate, SNCG protease conjugate, substrate solution, washing solution, and stop solution for detecting the concentration of the target protein.

25. The kit according to any one of claims 22-24, further comprising: instructions.

26. Use of the composition according to any one of claims 1-10 or the oligonucleotide according to any one of claims 11-19 in the preparation of a kit for in vitro detecting esophageal cancer.

27. The use according to any one of claims 20-21 and 26, wherein the kit for in vitro detecting esophageal cancer is used to detect esophageal cancer by a method including the following steps:
1) separating a DNA sample comprising the target sequence of a target gene or a fragment thereof from a biological sample to be tested;
2) determining the methylation state of the target sequence of the target gene; and
3) judging the state of the biological sample through the detection result of the methylation state of the target sequence of the target gene and/or the concentration of the target protein, so as to achieve in vitro detecting esophageal cancer.

28. The use according to claim 27, wherein the method includes the following steps:
extracting the genomic DNA of the biological sample to be tested;
treating the extracted genomic DNA with a reagent to convert the 5-position unmethylated cytosine base into uracil or other bases;
contacting the DNA sample treated with the reagent with DNA polymerase and the primer of the target sequence of the target gene, and performing DNA polymerization reaction in the presence of a blocker preferentially binding to the target sequence in an unmethylated state;
detecting an amplification product with a probe; and
determining the methylation state of at least one CpG dinucleotide of the target sequence of the target gene based on whether the amplification product exists.

29. The use according to claim 27, wherein the method includes the following steps:
collecting peripheral blood of a subject, and separating plasma or serum;
extracting free DNA from the plasma or serum;
treating the extracted free DNA with a reagent to convert the 5-position unmethylated cytosine base into uracil or other bases;
contacting the DNA sample treated with the reagent with DNA polymerase and the primer of the target sequence of the target gene, and performing DNA polymerization reaction in the presence of a blocker preferentially binding to the target sequence in an unmethylated state;
detecting an amplification product with a probe;
determining the methylation state of at least one CpG dinucleotide of the target sequence of the target gene based on whether the amplification product exists; and
determining the concentration of SNCG in the plasma or serum through immune response by using SNCG antibody.

30. The use according to claim 28 or 29, wherein the reagent is a bisulfite reagent.

31. A method for detecting esophageal cancer, which includes the following steps:
separating a DNA sample comprising the target sequence of a target gene or a fragment thereof from a biological sample to be tested;
determining the methylation state of the target sequence of the target gene; and
judging the state of the biological sample through the detection result of the methylation state of the target sequence of the target gene, so as to achieve in vitro detecting esophageal cancer.

32. A method for detecting esophageal cancer, which includes the following steps:
extracting the genomic DNA of the biological sample to be tested;
treating the extracted genomic DNA with a reagent to convert the 5-position unmethylated cytosine base into uracil or other bases;
contacting the DNA sample treated with the reagent with DNA polymerase and the primer of the target sequence of the target gene, and performing DNA polymerization reaction in the presence of a blocker preferentially binding to the target sequence in an unmethylated state;
detecting an amplification product with a probe; and
determining the methylation state of at least one CpG dinucleotide of the target sequence of the target gene based on whether the amplification product exists.

33. A method for detecting esophageal cancer, which includes the following steps:
collecting a biological sample of a subject;
determining the methylation state of the target sequence of the target gene in the biological sample of the subject;
determining the concentration of the target protein in the biological sample of the subject; and
jointly judging whether the subject has esophageal cancer through the detection result of the methylation state of the target sequence of the target gene and the concentration of the target protein, so as to achieve in vitro detecting esophageal cancer.

34. A method for detecting esophageal cancer, which includes the following steps:
collecting peripheral blood of a subject, and separating plasma or serum;
extracting free DNA from the plasma or serum;
converting the 5-position unmethylated cytosine base into uracil or other bases;
contacting the DNA sample treated with the reagent with DNA polymerase and the primer of the target sequence of the target gene, and performing DNA polymerization reaction in the presence of a blocker preferentially binding to the target sequence in an unmethylated state;
detecting an amplification product with a probe;
determining the methylation state of at least one CpG dinucleotide of the target sequence of the target gene based on whether the amplification product exists; and
determining the concentration of SNCG in the plasma or serum through immune response by using SNCG antibody.

35. The method according to any one of claims 31-34, wherein the target gene is one or both of MT1A gene and EPO gene.

36. The method according to claim 35, wherein the target sequence of MT1A gene is shown in SEQ ID NO:1.

37. The method according to claim 35, wherein the target sequence of EPO gene is shown in SEQ ID NO:3.

38. The method according to claim 32 or 34, wherein the reagent is a bisulfite reagent.

39. The method according to claim 32 or 34, wherein the primer is:
a fragment of at least 9 nucleotides in SEQ ID NO:1 or its complementary sequence and comprising at least one CpG dinucleotide sequence; and/or
a fragment of at least 9 nucleotides in SEQ ID NO:3 or its complementary sequence and comprising at least one CpG dinucleotide sequence.

40. The method according to claim 32 or 34, wherein the blocker is a blocker preferentially binding to the target sequence in an unmethylated state.

41. The method according to claim 32 or 34, wherein the probe is:
a fragment hybridizing to at least 15 nucleotides in SEQ ID NO:1 or its complementary sequence under moderately stringent or stringent conditions and comprising at least one CpG dinucleotide sequence; and/or
a fragment hybridizing to at least 15 nucleotides in SEQ ID NO:3 or its complementary sequence under moderately stringent or stringent conditions and comprising at least one CpG dinucleotide sequence.

42. The method according to claim 39, wherein the primer is the sequence of SEQ ID NO:5 and SEQ ID NO:6, or the sequence of SEQ ID NO:9 and SEQ ID NO:10.

43. The method according to claim 40, wherein the blocker is the sequence of SEQ ID NO:8, or the sequence of SEQ ID NO:12

44. The method according to claim 41, wherein the probe is the sequence of SEQ ID NO:7, or the sequence of SEQ ID NO:11.


SEQUENCE LISTING

<110> Biochain (Beijing) Science & Technology, Inc.

<120> COMPOSITION FOR DETECTING ESOPHAGEAL CANCER AND USE THEREOF

<130> BET 20M3268

<140> EP 18882701.8
<141> 2018-09-05

<150> CN201711248825.5
<151> 2017-12-01

<150> CN201810989986.8
<151> 2018-08-28

<160> 13

<170> PatentIn version 3.5

<210> 1
<211> 171
<212> DNA
<213> Artificial sequence

<220>
<223> Target sequence of MT1A gene

<400> 1
cacccagggg agctcagtgg actgtgcgcc ttgcctttct gctgcgcaaa gcccagtcca 60

ggtcatcacc tcgggcgggg cggactcggc tgggcggact cagcggggcg ggcgcaggcg 120

cagggcgggt cctttgcgtc cggccctctt tcccctgacc ataaaagcag c 171

<210> 2
<211> 171
<212> DNA
<213> Artificial sequence

<220>
<223> Complementary sequence of SEQ ID NO1

<400> 2
gctgctttta tggtcagggg aaagagggcc ggacgcaaag gacccgccct gcgcctgcgc 60

ccgccccgct gagtccgccc agccgagtcc gccccgcccg aggtgatgac ctggactggg 120

ctttgcgcag cagaaaggca aggcgcacag tccactgagc tcccctgggt g 171

<210> 3
<211> 259
<212> DNA
<213> Artificial sequence

<220>
<223> Target sequence of EPO gene

<400> 3
cgcgcacgca cacatgcaga taacagcccc gacccccggc cagagccgca gagtccctgg 60

gccaccccgg ccgctcgctg cgctgcgccg caccgcgctg tcctcccgga gccggaccgg     120

ggccaccgcg cccgctctgc tccgacaccg cgccccctgg acagccgccc tctcctccag     180

gcccgtgggg ctggccctgc accgccgagc ttcccgggat gagggccccc ggtgtggtca     240

cccggcgcgc cccaggtcg                                                  259

<210>  4
<211>  259
<212>  DNA
<213>  Artificial sequence

<220>
<223>  Complementary sequence of SEQ ID NO3

<400>  4
cgacctgggg cgcgccgggt gaccacaccg ggggccctca tcccgggaag ctcggcggtg      60

cagggccagc cccacgggcc tggaggagag ggcggctgtc cagggggcgc ggtgtcggag     120

cagagcgggc gcggtggccc cggtccggct ccgggaggac agcgcggtgc ggcgcagcgc     180

agcgagcggc cggggtggcc cagggactct gcggctctgg ccgggggtcg gggctgttat      240

ctgcatgtgt gcgtgcgcg                                                  259

<210>  5
<211>  16
<212>  DNA
<213>  Artificial sequence

<220>
<223>  MT1A primer F

<400>  5
cggacgtaaa ggattc                                                      16

<210>  6
<211>  20
<212>  DNA
<213>  Artificial sequence

<220>
<223>  MT1A primer R

<400>  6
gaaacgaact cgactaaacg                                                  20

<210>  7
<211>  19
<212>  DNA
<213>  Artificial sequence

<220>
<223>  MT1A probe

<400>  7

tgcgtttgcg ttcgtttcg                                                    19

<210>  8
<211>  33
<212>  DNA
<213>  Artificial sequence

<220>
<223>  MT1A blocker

<400>  8
caaactcaac taaacaaact caacaaaaca aac                                   33

<210>  9
<211>  20
<212>  DNA
<213>  Artificial sequence

<220>
<223>  EPO primer F

<400>  9
agtcgtagag tttttgggtt                                                  20

<210>  10
<211>  15
<212>  DNA
<213>  Artificial sequence

<220>
<223>  EPO primer R

<400>  10
caacgcgata cgacg                                                       15

<210>  11
<211>  16
<212>  DNA
<213>  Artificial sequence

<220>
<223>  EPO probe

<400>  11
cgcaacgaac gaccga                                                      16

<210>  12
<211>  30
<212>  DNA
<213>  Artificial sequence

<220>
<223>  EPO blocker

<400>  12
gagtttttgg gttattttgg ttgtttgttg                                       30

<210>  13
<211>  127
<212>  PRT
<213>  Artificial sequence

<220>
<223>  Amino acid sequence of a protein encoded by SNCG gene

<400>  13

Met Asp Val Phe Lys Lys Gly Phe Ser Ile Ala Lys Glu Gly Val Val
1               5                   10                  15

Gly Ala Val Glu Lys Thr Lys Gln Gly Val Thr Glu Ala Ala Glu Lys
            20                  25                  30

Thr Lys Glu Gly Val Met Tyr Val Gly Ala Lys Thr Lys Glu Asn Val
        35                  40                  45

Val Gln Ser Val Thr Ser Val Ala Glu Lys Thr Lys Glu Gln Ala Asn
    50                  55                  60

Ala Val Ser Glu Ala Val Val Ser Ser Val Asn Thr Val Ala Thr Lys
65                  70                  75                  80

Thr Val Glu Glu Ala Glu Asn Ile Ala Val Thr Ser Gly Val Val Arg
                85                  90                  95

Lys Glu Asp Leu Arg Pro Ser Ala Pro Gln Gln Glu Gly Glu Ala Ser
            100                 105                 110

Lys Glu Lys Glu Glu Val Ala Glu Glu Ala Gln Ser Gly Gly Asp
        115                 120                 125